# EUROPEAN PATENT APPLICATION

(11) **EP 2 324 869 A2**
(43) Date of publication of application: **25.05.2011**
(21) Application number: 10179576.3
(22) Date of filing: 07.11.2005
(51) Int. Cl.: A61M 1/10

(54) **Apparatus and method for direct organ perfusion**

(30) Priority: 07.11.2004 IL 16506804
(62) Divisional of application: 05803165.9
(71) Applicant: Drops Ltd, 38100 M.P. Hefer (IL)
(72) Inventor: Gildoni, M. Zvi, Ramat-Gan (IL); Hirszowicz, Eran, 52236 Ramat-Gan (IL)
(74) Representative: Vossius & Partner

(57) **Abstract**

The invention provides a balloon catheter comprising an elongated conduit adapted for insertion into a blood vessel, two or more balloons associated with said elongated conduit, and one or more additional conduits connecting the interior of two or more of said balloons, wherein said elongated conduit comprises two inflation/deflation channels which are suitable for transferring an inflation medium into and out of the balloons, and wherein said balloons and conduits are arranged such that said balloons may be inflated and deflated in a predetermined sequence.

## Description

### Field of the Invention

The present invention relates to an apparatus and method for direct organ perfusion. More particularly, the invention relates to a method and apparatus for carrying out renal perfusion for preserving and/or restoring renal function in case of renal failure or renal insufficiency and/or clinical conditions leading to renal failure and/or fluid overload.

### Background of the Invention

Acute renal insufficiency (ARI) sometimes also referred to as acute renal failure (ARF) is a clinical condition in which the kidneys function is impaired due to a disease, trauma, drugs or toxics that can damage the normal renal function. ARI can lead to a permanent loss of renal function. However, in certain cases, such kidneys can recover. Renal Failure (ARF) is a multi-factorial clinical syndrome characterized by rapid deterioration of renal function over hours or days, impairment of fluid and electrolyte metabolism, and accumulation of nitrogenous wastes.

ARI and ARF occurs in 1-5% of all hospitalized patients, but is more frequent in patients admitted to the Intensive Care Unit (ICU), where the occurrence rates of such problems can be about 30%. In-hospital mortality rate for ARI and ARF patients is about 50% and may be as high as 80% in ICU settings (Solucient 100 Top Hospitals® - 100 Top Hospitals: National Benchmarks for Success Class of 2002. Sept. 29, 2003, http://www.100tophospitals.com/Studiss/icu00/findings.asp).

This alarmingly high mortality of patients due to ARI and ARF remains unchanged during the last 40 years. About 70% of all ARF/ARI cases are attributed to compromised blood flow to the kidneys which is also referred at as "Pre Renal Failure".

The increased arterial resistance in CHF (Chronic or Congestive Heart Failure) patients, combined with hypotension present in the later stage of CHF, results in significant renal hypo-perfusion, which impairs renal function and in turn causes salt and water retention leading to signs and symptoms, which further contribute to CHF progression. Impaired renal function in CHF patients may even worsen further during drug therapy. Latest research indicates that mortality, morbidity and length of hospital stay are increased in patients where renal function deteriorates, a condition known as Aggravated Renal Dysfunction (ARD). It is now appreciated that the prevention of ARD during the treatment of heart failure may lead to better outcomes (Weinfeld, MS, et al., American Heart Journal; 1999; 138; pp 285-90).

The major risk factor of ARI and ARF patients is the decrease of effective arterial volume and hypotension. The current treatment for ARI and ARF patients, involves extended hospital stays, intravenous drug administration and in some cases hemofiltration and/or hemodialysis. Many of these patients are however too unstable (hemodynamically) to tolerate such treatment modalities.

Hemodialysis is a technology employing an extra-corporal circulation to remove solutes by diffusion. This measure is widely spread in handling chronic renal failure and may also be used for acute cases, leading to kidney transplant or to an on going chronic treatment, after accepting the fact that a concerned kidney will never function properly. This modality is however inefficient for solutes of high molecular weight that could be rather toxic, it may present a risk of hyperlipidaemia as well as a serious issue in controlling blood pressure, which may rule out this treatment for many CHF patients.

Hemofiltration is also an extra-corporal circulation based system. It removes solutes by convection and remains more constant for all solutes able to cross the semi-permeable membrane. However, hemofiltration is a slow, cumbersome, and a relatively costly process, prone to critical complications and requires time and additional personnel to attend the equipment. It can however only reduce, not normalize, concentration of larger solutes and since it is a rather long process it might be too long for patients in their acute phase, where a faster reaction is required. Although hemofiltration is used in the critical care setting, it is rarely used for CHF patients. Data indicate that more than half of the patients who have been on temporary hemodialysis may develop chronic renal failure.

Vasodilators, such as nitroglycerin and ACE (Angiotensin Converting Enzyme) inhibitors, are chemical agents that act directly on muscles in the blood vessel walls to dilate these vessels, by relaxing their muscular walls. By dilating the arteries, these agents allow blood to flow through more easily, reducing blood pressure and also allowing an easier path for restricted blood flow. Such agents are able to increase urinary sodium and water excretion even in certain conditions of moderate impairment of renal function, where it could be useful in alleviating course of acute renal failure. Such effect however may be obtained when vasodilators are used within 18 hours after ischemic and/or toxic event. Some vasodilators could also help in preventing ARF. It is however well known that this treatment can actually work only to a certain extent of renal ischemia and that it can eventually restore renal function only in cases of mild ischemia over a rather short period of time.

Other than the well recognized and unwanted side effects (e.g., chest pain and/or fast or irregular heartbeat; Headache, numbness or fainting; nausea or vomiting) of the available vasodilators, very poor cardiac outputs may not enable proper renal function despite the presents of rather excessive dosage of these drugs even if locally introduced into the renal arteries.

Direct Renal Perfusion (DRP) may be used to restore renal hemodynamics, and thereby improve kidney function and provide additional time for the heart to recover in cases of CHF. DRP methods usually utilize a catheter device to isolate a region of the aorta and direct that occluded volume to the desired organ (e.g. kidney) - perfusing vessels. There are also methods based on the withdrawal of the patient blood from one blood vessel, and being transferred via a catheter into the patient's renal arteries. Since DRP physically assists the kidney function, it takes off fluid overload, and thus allows the heart to work under less constrained conditions in cases of CHF (congestive heart failure).

DRP can prevent progression to the advanced stages of ARF by: restoring renal perfusion protecting the kidneys, reversing renal vasoconstriction, and increasing urine output. When applied to ICU patients, DRP reduces mortality, morbidity and costs associated with hypotensive episodes that would otherwise present high risk for renal failure and death. When applied to the CHF population, DRP therapy restores urine production and reduces fluid overload (which would restore hemodynamic balance), preserve the kidneys and reduce mortality, morbidity and costs associated with treatment of CHF patients.

Several DRP and related methods have been described in the prior art. US 5,505,701 describes an intra-aortic catheter which is inserted into the renal artery. The catheter consists of two occluding balloons located at the distal and proximal sides of a tube, and a permeable zone located between said balloons. The distal and proximal balloons are inflated separately via dedicated channels, in order to obstruct blood circulation in a region of the aorta and carrying out perfusion of fluid through the permeable zone. Thus, it requires three different channels (lumens) for carrying out the process, which imposes various restrictions on the catheter's dimensions, complicates its design and requires a designated complex device (special pump and control console), not yet described, in order to drive the said embodiment.

US 6,468,200 describes a multiple-chamber balloon catheter in which the chambers are inflated/deflated sequentially via a single lumen. The sequential inflation/deflation of the balloons is obtained by providing the balloons with apertures of different sizes, wherein the distal balloon is provided with the greatest sized aperture and the respective chambers are sized from larger to progressively smaller toward the proximal balloon. While this balloon catheter could be favorable as an intra-aortic balloon pump, it is not suitable for other organ/tissue perfusion.

A balloon catheter for carrying out perfusion to the renal arteries is described in US 2004/064091, US 2004/064090 and US 2002/0169413. The catheter described in these publications comprises two occluding balloons and an outlet port or a center balloon, disposed therebetween, for carrying out perfusion. These balloon catheters require one lumen for inflation/deflation of the occluding ballo ons and an additional lumen for carrying out perfusion via the center balloon or the outlet port. In addition, special consideration is required in this design in order to provide proper synchronization between inflations/deflations of the occluding balloons and the outlet port or center balloon performing the perfusion.

US 4,902,272 and US 6,210,318 describe balloon catheters in which a multi-lumen catheter is used for inflation/deflation of the balloons. These catheters are not suitable for carrying out perfusion of organs, such as the kidneys, and their design imposes limitations for such implementations due to their multi-lumen configuration.

It is an object of the present invention to provide a method and apparatus for direct organ perfusion, by means of a directional blood flow, generated by volume displacement within the blood vessels (no extra corporal circle employed). Such volume displacement is achieved by a number of balloons or different sizes, being inflated and deflated in a particular sequence. Said invention is utilizing a single inflation/deflation channel (just one of the catheter lumens) to maintain a sequential inflation/deflation of more than one balloon.

It is another object of the present invention to provide a method and apparatus for direct organ perfusion for preserving and/or restoring renal function of patients in risk of renal failure, and/or fluid overload, and the like. It is a further object of the present invention to provide a minimally invasive low profile catheter enabling localized, direct renal perfusion without disrupting the blood flow in the vessels it is passing through and with no need for cannulating the renal arteries.

It is a yet further object of the present invention to provide methods and apparatuses for direct organ perfusion that overcome the problems and disadvantages of the prior art solutions.

Other objects and advantages of the invention will become apparent as the description proceeds.

### Summary of the Invention

It has now been found that it is possible to construct a balloon catheter comprising one or more balloons and one or more inflating/deflating conduits, such that one or more of said balloons, when inflated, will occlude or partially occlude the normal blood flow in a main vessel, and such that an additional balloon will, upon inflation, direct a volume of blood into side vessels supplying an organ or other body site. In order to achieve the desired main vessel occlusion and increased side vessel perfusion, a novel arrangement of two or more balloons and conduits is employed, such that a defined inflation/deflation sequence of each of the balloons is obtained.

The present invention is thus primarily directed to a balloon catheter comprising an elongated conduit adapted for insertion into a blood vessel, two or more balloons associated with said elongated conduit, and one or more additional conduits connecting the interior of two or more of said balloons, wherein said elongated conduit comprises a single inflation/deflation channel which is suitable for transferring an inflation medium into and out of the balloons, and wherein said balloons and conduits are arranged such that said balloons may be inflated and deflated in a predetermined sequence.

By means of the presently-disclosed and claimed arrangement, the inventors provide an apparatus and method for direct organ perfusion. It is to be noted that the term "perfusion" is used herein to refer to the mechanism by which a volume of blood is displaced due to balloon(s) inflation(s) into the target blood vessels supplying the target organ(s), and that the term displacement balloon is used herein to refer to such balloon(s).

In one aspect, the present invention is directed to a balloon catheter comprising an elongated conduit adapted for insertion into a blood vessel, **characterized in that** said elongated conduit comprises at least a single inflation/deflation channel, for one or more balloons, Wherein the distal opening of said elongated conduit opens into the interior of a proximal balloon which is affixed to the distal portion of said elongated conduit, and wherein a distal balloon is arranged in proximity to said proximal balloon, and wherein an additional conduit connects the interior of said proximal balloon and the interior of said distal balloon.

Optionally, the distal end of the elongated conduit is sealed and the balloons are attached to the said elongated conduit, and wherein the proximal balloon is inflated via one or more lateral openings provided on said elongated conduit

The terms "proximal" and "distal" are used herein in relation to the operator (and not with reference to structures that are anatomically proximal or distal). The term "proximal" is thus used hereinabove and hereinbelow to refer to elements of the catheter device which are located in relative proximity to the operator, and the term, "distal" is used herein to refer to elements of the catheter device the location of which is relatively distant from the operator. The term "inflation/deflation channel" is used herein to refer to a channel or lumen whose function is to allow passage of an inflating substance (i.e. gas or liquid - preferably an inert substance such as helium) from a pressure source, such as a pump, to one or more balloons, thus enabling (under different conditions) the inflation and deflation of said balloons.

In another aspect, the present invention is directed to a balloon catheter comprising an elongated conduit adapted for insertion into a blood vessel, **characterized in that** said elongated conduit comprises at least a single inflation/deflation channel, the distal opening of which opens into the interior of the proximal section of a two-section balloon, wherein said proximal section is affixed to the distal portion of said conduit, and wherein an additional conduit connects the interior of said proximal section with said distal section.

Optionally, the distal end of the elongated conduit is sealed and the balloon sections are attached to the said elongated conduit, and wherein the proximal balloon section is inflated via one or more lateral openings provided on said conduit.

The present invention further provides a balloon catheter comprising an elongated conduit adapted for insertion into a blood vessel, **characterized in that** said conduit comprises at least a single inflation/deflation channel, the distal opening of which opens into the interior of a proximal balloon which is affixed to the distal portion of said conduit, wherein the interior of said proximal balloon connects via a second conduit to the interior of a distal balloon, and wherein a central balloon is situated between, and in proximity to, said proximal and distal balloons, and wherein a third balloon connects the interior of said distal balloon and the interior of said central balloon.

The aforementioned second conduit may pass from the proximal balloon to the distal balloon by any convenient route. In a preferred embodiment, however, the second conduit passes through the interior of the central balloon.

The present invention also provides a balloon catheter comprising an elongated conduit adapted for insertion into a blood vessel, **characterized in that** said conduit comprises at least one inflation/deflation channel, the distal opening of which opens into the interior of a distal balloon which is affixed to the distal portion of said elongated conduit, wherein said distal balloon is arranged in proximity to a central balloon, and wherein said central balloon is arranged in proximity to a proximal balloon, and wherein said central and proximal balloons are affixed to the distal portion of said elongated conduit, and wherein said elongated conduit comprises a side opening connecting the lumen of said channel with the interior of said proximal balloon, and wherein an additional conduit connects the interior of said distal balloon and the interior of said central balloon.

The present invention is also directed to a balloon catheter comprising an elongated conduit adapted for insertion into a blood vessel, **characterized in that** said elongated conduit comprises one inflation/deflation channel, the distal opening of which opens into the interior of a proximal balloon which is affixed to the distal portion of said elongated conduit, wherein the interior of said proximal balloon connects via a second conduit to the interior of a distal section of a two-section balloon, wherein the proximal section of said two-section balloon is arranged in proximity to said proximal balloon, and wherein a third conduit connects the interior of said distal section and the interior of said proximal section.

The aforementioned second conduit may pass from the proximal balloon to the distal section of the two-section balloon by any convenient route. In a preferred embodiment, however, the additional conduit passes through the interior of the proximal section of the two-section balloon.

The present invention further provides a balloon catheter comprising an elongated conduit adapted for insertion into a blood vessel, **characterized in that** said elongated conduit comprises one inflation/deflation channel, the distal opening of which opens into the interior of a distal section of a distally placed two-section balloon, wherein the proximal section of said two-section balloon comprises a proximal section arranged in proximity to a proximal balloon, and wherein said proximal section of said two section balloon and said proximal balloons are affixed to the distal portion of said elnongated conduit, and wherein said elongated conduit comprises a side opening connecting said channel with the interior of said proximal balloon, and wherein an additional conduit connects the interior of said distal section with the interior of said proximal section of said two section balloon.

In another aspect, the present invention encompasses a balloon catheter comprising an elongated conduit adapted for insertion into a blood vessel, **characterized in that** said elongated conduit comprises two inflation/deflation channels, wherein a first channel connects to the interior of a proximal balloon and a second channel connects to the interior of a distal balloon, and wherein an additional conduit connects the interior of said distal balloon and the interior of a central balloon situated between, and in proximity to, said proximal and distal balloons, and wherein said balloons are affixed to the distal portion of said elongated conduit.

The present invention is further directed to a balloon catheter comprising an elongated conduit adapted for insertion into a blood vessel, **characterized in that** said elongated conduit comprises two inflation/deflation channels, wherein a first channel connects to the interior of a proximal balloon and a second channel connects to the interior of a distal section of a distally placed two-section balloon, and wherein the proximal section of said two-section balloon is arranged in proximity to said proximal balloon, and wherein an additional conduit connects the interior of said distal section and the interior of said proximal section, and wherein said balloons are affixed to the distal portion of said elongated conduit.

In one preferred embodiment of this aspect of the invention, at least one of the balloons is manually inflated via a dedicated inflation channel. Although the manual inflation may be achieved by any suitable means, in a preferred embodiment, said inflation is accomplished with the use of an appropriate-sized separate inflation device such as a syringe.

In some specific preferred embodiments of the invention the distal balloon is arranged such that there is a gap between said distal balloon and said central balloon.

In another specific preferred embodiment of the invention the proximal balloon is arranged such that there is a gap between said proximal balloon and said central balloon.

Optionally, the displacement balloon is enclosed in the interior of a hollow confining element (e.g., open-ended mesh-like cage) comprising side apertures.

In another specific preferred embodiment of the invention the distal tip of the elongated conduit extends beyond the distal end of the distal balloon, and the interior of said distal balloon is connected to the interior of said elongated conduit via a side opening, and wherein said balloon catheter further comprises an additional lumen and a stiffening wire within said lumen

The distal end section of the stiffening wire may be manufactured from an elastic material in a coiled shape, such that when said distal end section of said wire exits the passage in which it is contained via a distal opening of said passage, the coiled shape of said wire is restored

In a further aspect, the present invention also encompasses a method for increasing blood flow to one or more organs/areas in a subject, comprising the steps of:
a) inserting a balloon catheter into a blood vessel and advancing said catheter through the vasculature until it is placed in a main blood vessel in proximity to a specific organ or tissue or to the branch vessels supplying said organs, wherein said balloon catheter comprises an elongated conduit having an inflation/deflation channel, for the passage of an inflation or deflation media (fluid or gas), the distal opening of which opens into the interior of a proximal balloon which is affixed to the distal portion of said elongated conduit, and wherein a distal balloon is arranged in proximity to said proximal balloon, and wherein an additional conduit connects the interior of said proximal balloon and the interior of said distal balloon;
b) expanding said proximal balloon by means of a pump/console device connected to the proximal end of a lumen of said catheter allowing the passage of said shuttle gas or fluid, such that the expanded proximal balloon occludes or partially occludes said main blood vessel,;
c) allowing said distal balloon to expand as a result of the passage of fluid (or gas) via the additional conduit internal space, such that the expanding distal balloon displaces a volume of blood into said branch vessels;
d) sequentially deflating said proximal balloon and said distal balloon by causing said pump to evacuate the fluid (or gas) therefrom; and
e) repeating steps b) to d), and optionally coordinating the expansion and deflation of the balloons with the cardiac cycle (or other signal) of said subject, wherein the operation of said pump is regulated by control means connected thereto.

The present invention also provides other methods for increasing blood flow to one or more organs/directions in a subject, similar to that disclosed immediately hereinabove, wherein in each of said methods, the arrangement of balloons and conduits is in accordance with the various balloon catheters disclosed hereinabove, and described in more detail hereinbelow.

### Brief Description of the Drawings

In the drawings:
- Figs. 1A-1C illustrates an implementation of the balloon catheter of the invention consisting of two separate balloons;
- Figs. 2A-2C illustrate another implementation of the invention in which the balloon catheter consists of three separate balloons;
- Fig. 3 illustrates another preferred embodiment of the three-balloon catheter of the invention in which the interior of the occluding balloons are connected directly via the inflation/deflation channel/s of the catheter;
- Fig. 4 illustrates an embodiment in which a single balloon consisting of two connected sections is used for implementing the two-balloons catheter;
- Fig. 5A-5B illustrates an embodiment in which a single balloon consisting of two connected sections and an additional balloon is used for implementing the three-balloons catheters of the invention;
- Fig. 6 demonstrates an alternative embodiment of the three-balloon catheter of the invention in which the proximal occluding balloon is inflated via a separate lumen utilizing a separate inflation device such as a syringe;
- Fig. 7 demonstrates an alternative embodiment in which a single balloon consisting of two connected sections and an additional balloon are used for implementing the three-balloons catheters of the invention wherein the proximal occluding balloon is inflated via a separate lumen utilizing a separate inflation device such as a syringe;
- Fig. 8 depicts a general embodiment of the invention wherein a sequence of numerous balloons are arranged to achieve any sequence of balloon inflations/deflations like such which may start from the distal and proximal balloons and proceeds via intermediate balloons towards the center of the balloon arrangement;
- Fig. 9 illustrates another preferred embodiment of the three-balloon catheter of the invention wherein there is a gap between the distal balloon and the displacement balloon;
- Fig. 10 illustrates another preferred embodiment of the three-balloon catheter of the invention wherein the displacement balloon is enclosed in a confining element;
- Fig. 11 illustrates another preferred embodiment of the three-balloon catheter of the invention wherein the catheter comprises a lumen for an accessory wire;
- Fig. 12 illustrates another preferred embodiment of the three-balloon catheter of the invention wherein there is a gap between the proximal balloon and the displacement balloon;
- Fig. 13 illustrates a specific preferred embodiment of the balloon catheter of the invention comprising two separate balloons that are attached to the catheter shaft;
- Fig. 14 illustrates a specific preferred embodiment of the balloon catheter of the invention comprising a single balloon consisting of two connected sections that are attached to the catheter shaft.

### Detailed Description of Preferred Embodiments

The use of DRP (Direct Renal Perfusion - but also related to other possible applications such as heart support, liver, limb, etc.) of the invention will allow specific subsets of patients to recover from a variety of conditions while diminishing or avoiding the risks of fetal or chronic renal damage. In addition, DRP treatment of the invention can be used in conjunction with current drug and fluid therapies. The balloon catheter can be introduced and activated at bedside and remain indwelling for the duration of the treatment. It can be also placed while in the emergency room and during resuscitation, allowing the medical team to treat the renal system in isolation from other organs (for example, permitting pulmonary edema to be controlled without increasing renal insufficiency).

Moreover, the balloon catheter of the invention is designed to complement with other known devices, already available on the market, such as Intra Aortic Balloon, equally suitable Cardiac Support Devices or different interventional diagnostic or therapeutic means. Therefore it can be designed to be complementary, rather than competitive, with other devices which are already being used in ICUs and other hospitalization facilities. The simple small sized indwelling balloon element will not interfere with other treatment modalities, including local drug delivery or required percutaneous interventions.

The perfusion apparatus of the invention consists of a balloon catheter designed for direct organ perfusion and specifically for carrying out DRP to patients at risk of ARI/ARF and to CHF patients or others with fluid overload and impaired renal functions. A novel balloon catheter design is used, which enables localized perfusion to the renal arteries with minimal disruption of regular blood flow and without requiring renal cannulation.

In DRP applications, for example, the balloon catheter device of the invention is to be placed at the lumbar level of the abdominal aorta, close to the renal arteries bifurcation. The catheter is composed of two primary components, an inflatable occluding element and a blood displacement (perfusion) element. The obstruction mechanism is preferably comprised of one or more balloons. The blood displacement element is preferably also a balloon made of a similar material (e.g., polyurethane) (though of different size). The balloons are connected to a shaft at the distal end of the catheter, wherein said shaft is preferably of a 5-7 French in diameter. The catheter is designed to operate with a single inflation/deflation channel utilizing a suitable driving gas or fluid as is well known in the art (e.g., Helium) and a pump device connected to the proximal end of the catheter.

The term "French" (short for French Gauge) refers to a measurement unit commonly used for grading tubes circumferences, wherein 1 French is equivalent to 1/Π millimeter (i.e., 1 mm ≈ 3.1416 Fr).

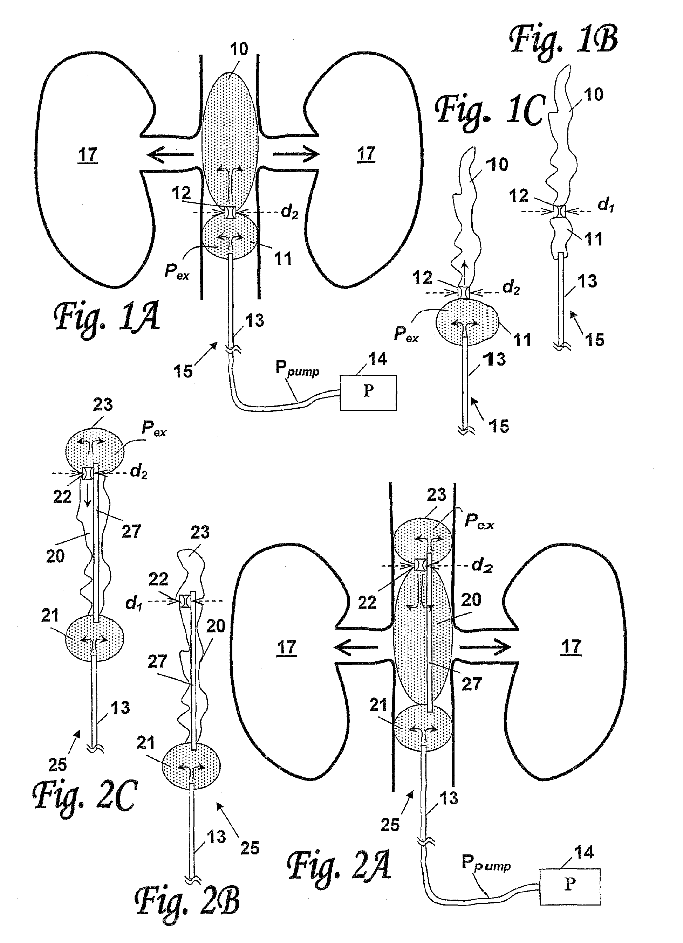

Figs. 1A-1C illustrates a preferred embodiment of the invention in which the balloon catheter **15** consists of two separate balloons. The proximal balloon **11,** is an occluding balloon connected at the distal end of catheter shaft **13** to the inflation/deflation channel that is used for inflation/deflation of the balloons via pump device **14.** The interior of the proximal balloon **11** is connected to the interior of the displacement balloon **10** via slender-passage element **12.** The balloon catheter **15** is inserted in a deflated state (Fig. 1B) via a peripheral (typically the femoral) artery, and advanced until its distal tip is located in situ near the target organ **17,** In DRP procedures for instance, the balloon catheter **15** is preferably inserted via a femoral artery and advanced up toward the bifurcation leading to the target organ **17.** Fig. 1A demonstrates a renal perfusion procedure in which the displacement balloon **10** is disposed adjacent to the renal arteries.

After placing the balloon catheter **15** the proximal balloon **11** is inflated by pump device **14,** via the inflation/deflation channel of catheter shaft **13.** When the balloons are deflated, the diameter of the passage *d₁* of the slender-passage element **12** enables leakage of a small amount of the inflation gas (or liquid) to pass from the proximal balloon **11** to the displacement balloon **10.** Therefore, the proximal balloon **11** is inflated first while the displacement balloon **10** remains deflated, as shown in Fig. 1C. The proximal balloon is inflated until its sides are pressed against the aortic (or other main vessel) wall, thereby momentarily obstructing (or reducing) the normal blood flow. At this stage the further inflation of the proximal balloon **11** cause a build-up of an excess pressure *Pₑₓ* in the proximal balloon which exceeds a threshold pressure *Pₜₕ* (i.e., *Pₑₓ*>*Pₜₕ*) which causes the internal space of slender-passage element **12** to expand to diameter *d₂* (*d₂*>*d₁*), which thus permitting rapid inflation of the displacement balloon **10,** as shown in Fig. 1C.

In this way, inflation of the proximal balloon **11** causes a build-up of pressure at the bifurcation area. Inflation of the displacement balloon **10** while the main vessel (aorta) is being occluded (or partially occluded) by inflated balloon 11, forces the blood accumulated around the balloon area to flow into the target vessel (organ) **17.** Balloons inflation/deflation is preferably synchronized with the ECG or blood pressure waveforms signals of the patient such that the inflation and deflation of the balloon occur at the optimal point of each cycle (such as inflation during diastole and deflation of the balloons shortly before ventricular systole).

Each inflection and deflation of the entire balloons configuration could be programmed to last, for example, the time equivalent to 1 heart cycle (about 0.75 seconds at a 80BPM heart rate) namely about 0.3Sec - 0.6Sec for each inflation or deflation. At certain situations programming one inflate/deflate cycle every second or third (or more) heart beat could also be considered.

Changing the operation of the pump device **14** into the deflation mode first deflates the occluding balloon (or balloons) **11** followed by deflation of the displacement balloon **10.** This deflation sequence is achieved by virtue of the design of the single inflation/deflation channel balloon catheter **15,** and is particularly advantageous since it prevents suction of the enforced blood from the perfused arteries back into the main vessel (aorta), which might have occurred if the displacement balloon **10** was first to deflate.

This inflation/deflation sequence may be similarly achieved by implementing a slender-passage element **12** with a narrow passage conduit (also termed nozzle), which induces a delay in the inflation/deflation of displacement balloon **10.** In such implementation, during the inflation, relatively small portions of the inflation media (e.g., fluid) filling the occluding balloon **11** also travel into displacement balloon 10 via slender-passage element **12.** However, due to the narrow passage of slender-passage element **12** connecting the interiors of the balloons, and since the volume of displacement balloon **10** is greater than the volume of occluding balloon **11,** the interior of the occluding balloon is filled will the inflation media faster.

Similarly, during deflation, relatively small portions of inflation media travels from the interior of the occluding balloon into displacement balloon **10** via slender-passage element **12.** The narrow passage provided by slender-passage element **12** and the volume difference between the balloons guarantees faster deflation of the occluding balloon **10.** Catheter **13** may optionally be a reinforced catheter and it is preferably made of a biocompatible material, such as polyurethane, Teflon, or PVC, its diameter is preferably between 4 to 18 French, and for DRP implementations preferably less than 7 French. Catheter shaft **13** preferably comprises a supporting wire (not shown) to support catheter insertion. The balloons are preferably made of non-compliant materials, although compliant materials can be used for certain applications, and they are preferably made of a biocompatible material, such as polyurethane.

In DRP implementations the length of the occluding balloon **11** in its inflated state is typically about 1 centimeter, its diameter is about 2 centimeters, and its volume is about 1cc. In its inflated state, the length of the displacement balloon **10** is about 6 centimeters, its diameter is about 1.4 centimeters, and its volume is about 10cc. These parameters are given, however, only by way of example and may be changed in accordance with the differing requirements of the various embodiments of the present invention. Thus, the abovementioned parameters should not be construed as limiting the scope of the present invention in any way.

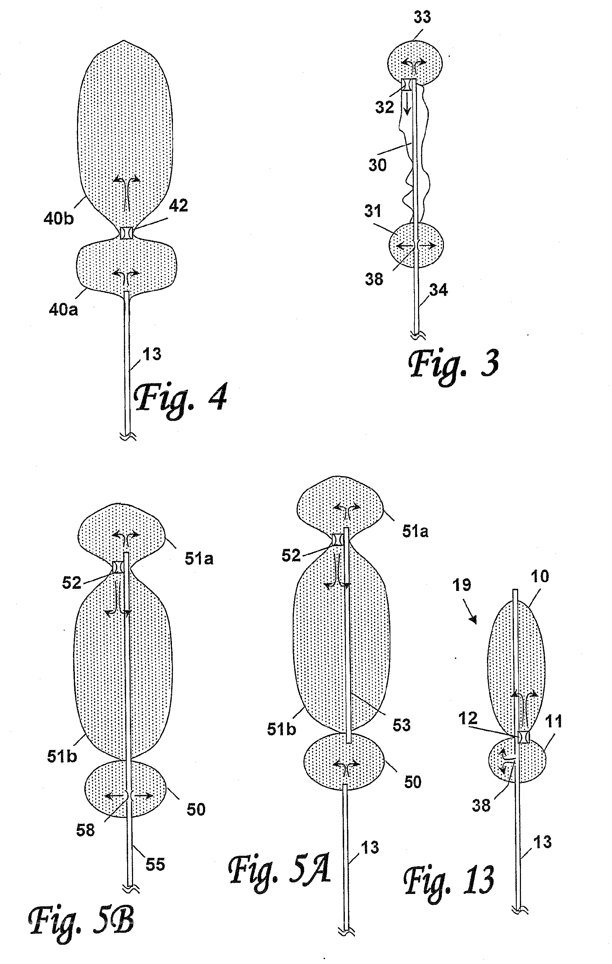

Fig. 13 shows a specific preferred embodiment of the balloon catheter shown in Figs. 1A-1C, wherein the separate balloons, **10** and **11,** of balloon catheter **19** are attached to the outer surface of catheter shaft **13.** The operation and structure of this preferred embodiment is substantially similar to the embodiments described with reference to Figs. 1A-1C. The main differences between these preferred embodiments are that the interior of occluding balloon **11** is connected to the inflation lumen of catheter shaft **13** via a lateral aperture **38** provided thereon, and thus in this case the distal tip of shaft **13** is sealed. Catheter shaft **13** preferably comprises a supporting wire (not shown) to support catheter insertion.

Another preferred embodiment of the invention is shown in Figs. 2A-2C, wherein the balloon catheter **25** consists of two occluding balloons, proximal balloon **21** and distal balloon **23,** and a displacement balloon **20** disposed between them. The lumen of the inflation/deflation channel of catheter **13** connects the interior of the proximal balloon **21** to the external pump device **14** and the lumen of a distinct conduit **27** connects the interior of the proximal balloon **21** to the interior of the distal balloon **23,** such that the driving gas (or liquid) can pass from the interior of the proximal balloon **21** into the interior of the distal balloon **23.** Said distinct conduit **27** is preferably situated within the displacement balloon **20.** A slender-passage element **22** connects the interior of the distal balloon **23** with the interior of the displacement balloon **20.**

The balloon catheter **25** illustrated in Figs. 2A-2C is inserted in a similar fashion, as was described herein above with reference to Figs. 1A-1C. Once it is placed in the desired location the pump device **14** can be activated, in order to inflate/deflate the occluding and displacement balloons **21, 23,** and **20.** During inflation, the inflation gas (or liquid) flows from the pump **14** via the lumen of the inflation/deflation channel of catheter **13** into the interior of proximal balloon **21,** and via the hollow conduit **27** into the interior of the distal balloon **23.** In the deflated state of the occluding balloons **21** and **23** the diameter of the passage *d₁* of the slender-passage element **22** enables a negligible portion of the driving gas (or liquid) to pass from the distal balloon **23** to the displacement balloon **20.** Therefore, the occluding balloons **21** and **23** are inflated first while the displacement balloon **20** remains deflated, as shown in Figs. 2B-2C. The occluding balloons **21** and **23** are inflated until their sides are pressed or put in proximity to the vessel walls and thereby obstruct (or increase resistance of) the blood flow up and/or downstream. This excess pressure build up in the occluding balloons **21** and **23** forces a wider diameter *d₂* of the slender-passage element **22,** which in result enable rapid inflation of the displacement balloon **20,** as shown in Fig. 2C.

Of course, a similar delay between the inflation/deflation of occluding balloons **23** and **21** and the inflation/deflation of the displacement balloon **20** may be achieved by implementing slender-passage element **22** by a nozzle as was previously explained hereinabove.

In this way, the blood trapped between the occluding balloons **21** and **23** is forced to flow into the target vessel toward the organ or area **17** upon inflation of the displacement balloon **20.** The inflation of the balloons is preferably synchronized with the ECG or blood pressure waveforms signals of the patient in order to optimize inflation/deflation timing (e.g. such that the inflation of the balloon occurs during diastole and deflation of the balloons shortly before ventricular systole).

Each inflation and deflation of the entire balloons configuration could be programmed, for example, to last the time equivalent to 1 heart cycle (about 0.75 seconds at a 80BPM heart rate) namely about 0.3Sec - 0. 6Sec for each inflation or deflation. At certain situations programming one Inflate/deflate cycle every second or third (or more) heart beat could also be considered.

Changing the operation of the pump into the deflation mode first deflates the occluding balloons **21** and **23** followed by deflation of the displacement balloon **20.** Similarly, this deflation sequence is achieved by virtue of the design of the single inflation/deflation channel of catheter **25,** and it advantageously prevents suction of the perfused blood form the perfused arteries back into the main vessel, which might have occurred if the displacement balloon **20** was first to deflate.

The conduit **27** connecting the interiors of the occluding balloons **21** and **23** are preferably made from a biocompatible material, such as polyurethane, Teflon, or PVC, its diameter preferably about 5 to 6 French. The balloons are preferably made of a biocompatible material, such as polyurethane. In DRP implementations the lengths of the occluding balloons **21** and **23** in their inflated state are typically about 1 centimeter, and their diameters are preferably about 2 centimeters. In its inflated state, the length of the displacement balloon **20** is about 6 centimeters, and its diameter is about 1.4 centimeter. These parameters are given, however, only by way of example and may be changed in accordance with the differing requirements of the various embodiments of the present invention. Thus, the abovementioned parameters are not to be construed as limiting the scope of the present invention in any way.

The pump device **14** can be any state of the art pump, utilizing gas (e.g., Helium) or liquid (e.g., water) for balloon inflation/deflation, and that can be controlled in correspondence with the ECG or blood pressure wave forms signals received from the patient, for example, by directly detecting such signals by the pump console or by other hospital standard monitoring devices such as the CS 100 Intra-Aortic Balloon Pump manufactured by Datascope Corp. of Montvale, NJ 07645 US, or the AutoCAT^{™} 2 WAVE^{™} IABP System by Arrow International Inc. of Reading, PA 19605 US.

The threshold pressures *Pₜₕ* and the pump pressure *Pₚᵤₘₚ* are preferably above normal blood pressure (∼140mmHg), but their values may vary according to the needs of the particular implementation. In the preferred embodiment of the invention the diameter of the slender-passage element in its contracted state is about 0.5 millimeters, and in its extracted state the diameter of said passage may vary between 0.5 millimeters to 3 millimeters. When implementing slender-passage element **12** by a slender passage conduit (nozzle), the internal diameter thereof is generally in the range of 1Fr to 8 Fr, preferably about 2Fr. These parameters are given, however, only by way of example and may be changed in accordance with the differing requirements of the various embodiments of the present invention. The slender passage elements may be made from any suitable biocompatible material. In a preferred embodiment, said elements are constructed from silicon. Thus, the abovementioned parameters are not to be construed as limiting the scope of the present invention in any way.

Fig. 3 illustrates another preferred embodiment of the three-balloon catheter of the invention in which the lumen of the inflation/deflation channel of catheter shaft **34** connects the interiors of the occluding balloons **31** and **33.** When inflated, the two occluding balloons are inflated simultaneously via the inflation/deflation channel of catheter shaft **34.** The proximal balloon **31** is inflated via aperture(s) **38** connecting the lumen of the inflation/deflation channel with the interior of balloon **31,** while the distal balloon **33** is inflated via the distal opening of the inflation/deflation channel. Slender-passage element **32** connecting the interiors of occlusion balloon **33** and of displacement balloon **30** induces a delay in the inflation of displacement balloon **30,** and thus guarantees that the displacement balloon **30** is inflated only after that the occluding balloons **31** and **33** are fully inflated, in a similar manner to the embodiments described hereinabove.

Catheter shaft **34** preferably comprises a supporting wire (not shown) to support catheter insertion.

Fig. 4 illustrates another embodiment of the two-balloon catheter of the invention. In this embodiment the balloon catheter consists of a single balloon comprising two sections, an occluding section **40a** and a displacement section **40b.** The interior of these sections is connected via a slender-passage element **42,** which guarantees that the displacement section **40b** is inflated only after the occluding section **40a** is fully inflated, in a similar manner to the embodiments described hereinabove.

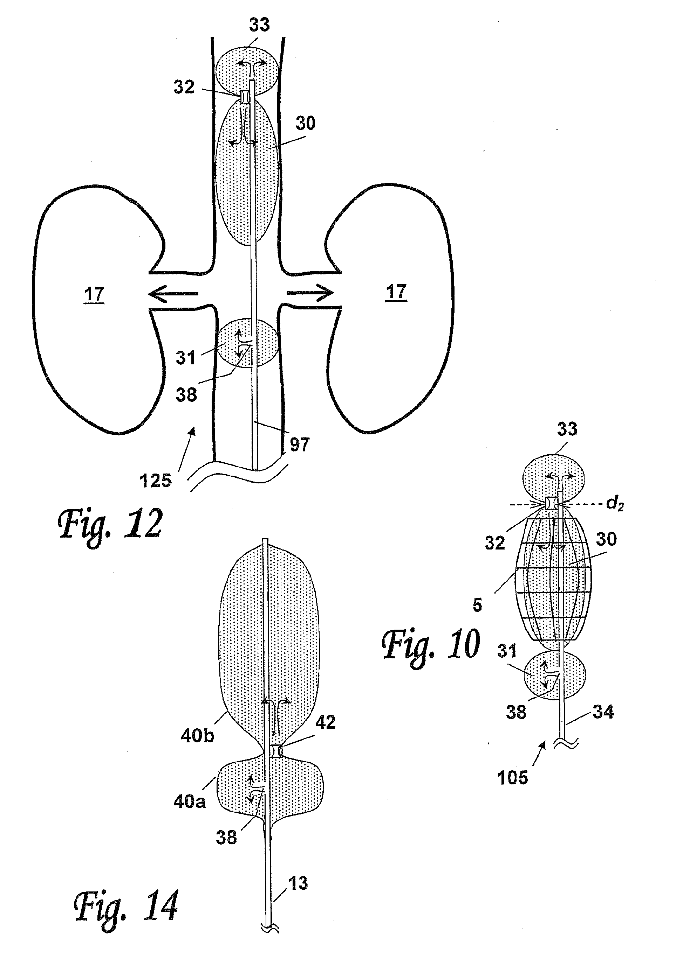

Fig. 14 shows a specific preferred embodiment of the balloon catheter shown in Fig. 4, wherein the balloon sections, **40a** and **40b,** of balloon catheter are attached to the outer surface of catheter shaft **13.** The operation and structure of this preferred embodiment is substantially similar to that of the embodiment described with reference to Fig. 4. The main differences between these preferred embodiments are that the interior of occluding section **40a** is connected to the inflation lumen of catheter shaft **13** via a lateral aperture **38** provided thereon, and thus in this case the distal tip of shaft **13** is sealed.

Catheter shaft **13** preferably comprises a supporting wire (not shown) to support catheter insertion.

Figs. 5A-5B depicts a variant of the three-balloon catheter of the invention, shown in Figs. 2A-2C, wherein the distal balloon and the displacement balloon are formed by a single balloon consisting of two sections, a displacement section **51b** and an occluding section **51a,** the interiors of which are connected via slender-passage element **52.** In the embodiment shown in Fig. 5A, a separate conduit **53** is used for connecting the interiors of the proximal balloon **50** and of the occluding section **51a.** In the embodiment shown in Fig. 5B, the lumen of the inflation/deflation channel of catheter 55 is connecting the interiors of the proximal balloon **50** and the distal section **51a,** and thus they are simultaneously inflated/deflated via apertures **58** connecting the interior of the distal balloon **50** with the lumen of the inflation/deflation channel of catheter **55,** and via the distal opening of the inflation/deflation channel at the distal end of catheter **55** connecting said channel to the interior of the occluding section **51a.** In both of these embodiments the displacement section **51b** is inflated only after the occluding section **51a** and the proximal balloon **50** are fully inflated, due to the slender-passage element **52,** the functioning of which is similar to the slender-passage element previously described herein.

It should be noted that although the preferred embodiments of the invention which were described hereinabove with reference to Figs. 5A and 5B were exemplified to include separate proximal balloon **50,** similar results can be achieved by utilizing a single balloon comprising distinct sections the interior of which are connected in a similar manner. Therefore, for instance, proximal occluding balloon **50** may be implemented as a proximal section of the main balloon (comprising sections **51a** and **51b).** Such a multiple sectioned balloon may be also used in all other embodiments of the invention.

Catheter shaft **55 (13)** may comprise a supporting wire (not shown) to support catheter insertion.

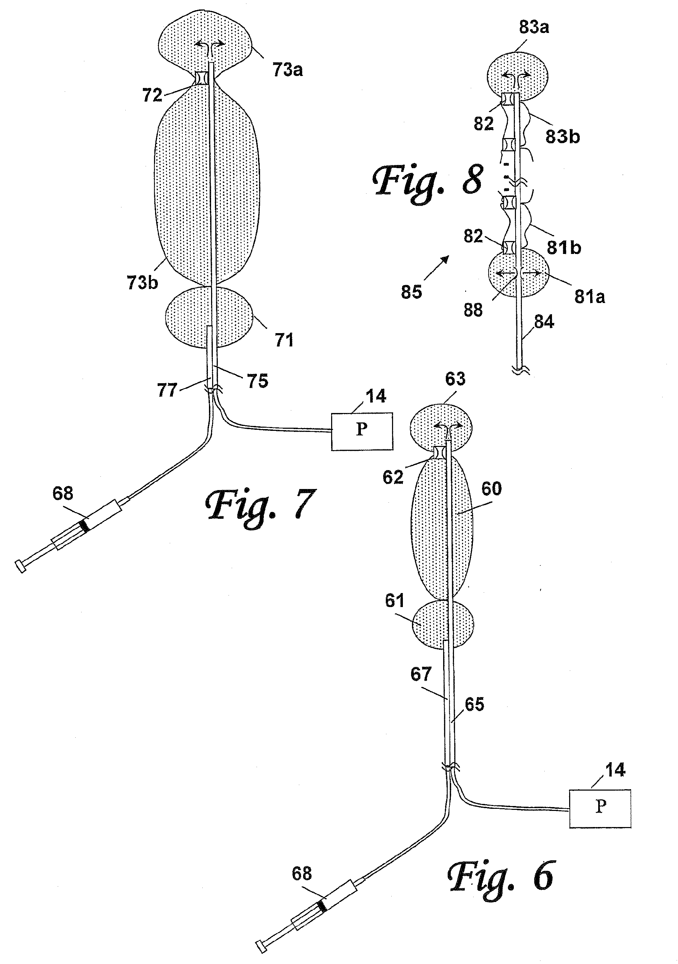

Fig. 6 demonstrates an alternative embodiment of the three-balloon catheter of the invention in which the proximal occluding balloon **61** is inflated via a separate inflation/deflation channel **67** e.g. by utilizing a separate inflation device such as a syringe **68.** In this embodiment of the invention the inflation/deflation of the distal occluding balloon **63** and of the displacement balloon **60** are carried out via the lumen of the inflation/deflation channel **65** connected to the pump **14.** The interiors of the distal occluding balloon **63** and of the displacement balloon **60** are connected via slender-passage element **62** which guarantees that the displacement balloon is inflated after inflation of the distal occlusion balloon **63.** In this way, the proximal occluding balloon **61** can be manually (or otherwise) inflated to partially obstruct the main vessel (e.g. aorta) such that part of the blood flow can pass on while in this state. The inflation/deflation of the distal occluding balloon **61** and of the displacement balloon **60** are preferably synchronized with the ECG or blood pressure waveform signals of the patient as was previously described.

Catheter shaft **65** preferably comprises a supporting wire (not shown) to support catheter insertion.

Fig. 7 demonstrates another alternative embodiment in which a single balloon consisting of two connected sections, **73a** and **73b,** and a separate proximal occluding balloon **71** are used for implementing the three-balloon catheter of the invention, and wherein the proximal occluding balloon **71** is inflated via a separate inflation/deflation channel **77** utilizing a separate inflation device such as a syringe **68.** In this embodiment of the invention the inflati on/deflation of the distal occluding section **73a** and of the displacement section **73b** are carried out via the lumen of inflation/deflation channel **75** connected to the device **14.** The interiors of the distal occluding section **73a** and of the displacement section **73b** are connected via slender-passage element **72** which guarantees that the displacement section is inflated after inflation of the distal occluding section. In this way, the proximal occluding balloon **71** can be manually inflated to obstruct or partially obstruct the main vessel (e.g. aorta) such that part of the blood flow can pass through while in this state. The inflation/deflation of the distal occluding section **73a** and of the displacement section **73b** are preferably synchronized with the ECG or blood pressure waveform signals of the patient as was previously described.

Catheter shaft **75** preferably comprises a supporting wire (not shown) to support catheter insertion.

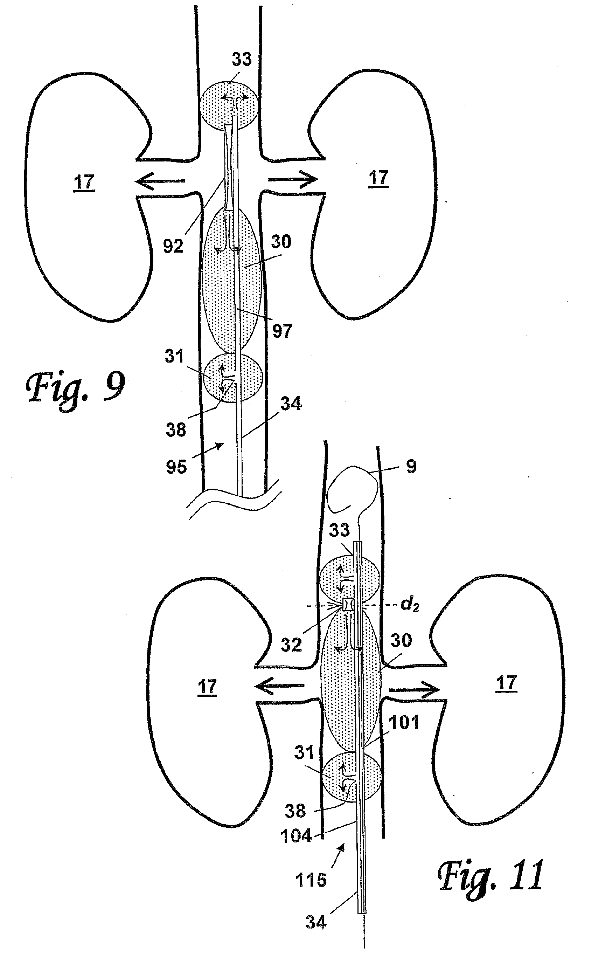

In order to reduce the risk of the balloon occluding the target vessel, either a compliant balloon (i.e., which substantially retains its shape) should be used or the balloon catheter structure of the invention modified by adding confining and/or supporting means, as will be now described with reference to Figs. 9-11.

Fig. 9 shows a preferred embodiment of the three-balloon catheter **95** of the invention wherein the distal balloon **33** is placed remote from the displacement balloon **30** thereby providing a gap between said balloons. This configuration of balloon catheter **95** allows placing it such that the displacement balloon **30** is located below the bifurcation, thereby preventing occlusion thereof by the sides of said balloon.

The gap between distal balloon **33** and displacement balloon **30** is preferably obtained by placing displacement balloon **30** and proximal balloon **31** proximally distant from the distal tip of catheter **34.** The dimensions of slender passage **92** should therefore be adjusted in order to deliver (and/or remove) inflation fluids/gas into (and/or from) displacement balloon **30** over this gap, and the location of aperture **38** should be also adjusted accordingly, in order to connect between the interiors of catheter **97** and of proximal balloon **31,** in its distant location. In this preferred embodiment slender passage **92** is prolonged according to the gap required between distal balloon **33** and displacement balloon **30.**

The operation of balloon catheter **95** is substantially similar to the operation of balloon catheters of the invention which were previously described hereinabove. The gap between distal balloon **33** and displacement balloon **30** is generally in the range of 80 mm to 120 mm, preferably about 100 mm. The length of prolonged slender passage **92** is thus generally in a range corresponding with the distance between said balloons.

Catheter shaft **34** preferably comprises a supporting wire (not shown) to support catheter insertion.

It should be noted that a similar gap may be similarly introduced in the various three-balloon catheter embodiments of the invention between the proximal balloon and the displacement balloon, by modifying them accordingly. Fig. 12 demonstrates such a preferred embodiment of the invention wherein there is a gap between the proximal balloon **31** and the displacement balloon **30** of balloon catheter **125.**

Fig. 10 illustrates another preferred embodiment of the three-balloon catheter **105** of the invention wherein the displacement balloon (or segment) **30** is enclosed in a confining element **5.** The structure and elements of balloon catheter **105** may be implemented according to any of the preferred embodiments which were previously described hereinabove. Confining element **5** is preferably made in a form of an open-ended mesh-like cage. Displacement balloon **30** is placed inside the hollow interior of confining element **5** end the catheter shaft **34** passes via its distal and proximal openings.

The enclosure of displacement balloon **30** in confining element 5 prevents the occlusion of the bifurcating blood vessel(s) by the displacement balloon **30.** Confining element may be manufactured from a biocompatible material, such as polyurethane, and should be designed properly to allow sufficient inflation space for displacement balloon **20.** For example, in one specific preferred embodiment of the invention the confining element **5** is equivalent in size (or somewhat over) to that of the fully inflated displacement balloon. Moreover, confining element **5** may be manufactured from a resilient material (e.g., silicon), thereby allowing it to expand and contract in accordance with the inflation and deflation of displacement balloon **30.**

Catheter shaft **34** preferably comprises a supporting wire (not shown) to support catheter insertion.

Fig. 11 demonstrates another preferred embodiment of the three-balloon catheter **115** of the invention wherein the catheter comprises a passage **101** for passing a stiffening wire **9.** Apart from adding in balloon catheter **115** a passage **101** for said stiffening wire **9,** the structure and elements of balloon catheter **115** may be implemented according to any of the preferred embodiments which were previously described hereinabove.

Stiffening wire **9** may be further adapted for centering balloon catheter **115** in the vessel (or cavity) wherein it is operated. Such functioning may be obtained by using an elastic (or less elastic) wire like such having a coil-shaped distal end section. During insertion of balloon catheter **115** wire **9** could remain in passage **101** such that its distal tip is maintained there inside. After placing balloon catheter **115** in the destined treatment site, the distal end section of wire **9** could be pushed distally such that it exits passage **101** via a distal opening provided at the distal end of catheter **115.** After exiting passage **101** the distal end section of wire **9** reverts to its coiled shape such that the lateral portions of its coiled structure are pressed against the wall of the vessel (or cavity), thereby centering wire **9** and catheter **115** therein.

Wire **9** may be manufactured from metal, such as Nitinol or stainless steel. Passage **101** may be provided externally (e.g., by attaching an auxiliary tube to the catheter body), or it may be integrated in the catheter body using conventional catheter manufacturing techniques. The length of the distal end portion of wire **9** in the case of DRP is preferably in the range of 10 mm to 30 mm, and the diameter of its coiled structure is generally in the range of 10 mm to 30 mm. Wire **9** may be produced using conventional wire manufacture methods.

The pump and control unit used in the present invention may be of any of the types known in the prior art, already available on the market, such as Intra Aortic Balloon Pumps or equally suitable Device. For example, such pumps are available these days from companies like Arrow international, Datascope Inc. and others, as indicated hereinabove.

Potential therapeutic targets for the new system are ICU patients who are at risk of Acute Renal Failure (septic shock, trauma, post-surgical complications, etc.), patients undergoing major vascular surgery (such as heart or abdominal aortic procedures) and Heart Failure patients at acute episodes of fluid overload, when refractive to medications and unable to establish fluid balance by standard treatment.

The perfusion apparatus of the invention will also be effective as a protective means in procedures likely to lead into ARI/ARF, such as diagnostic or therapeutic measures involving the administration of iodine-based dye contrast media, like coronary or peripheral angiograms, angioplasty and other minimally invasive interventions.

It should be noted that the use of the balloon catheter of the invention to increase the perfusion of a body organ may have the additional benefit of increasing blood flow to the brain of the treated subject.

As was described in details hereinabove the per fusion system of the invention uses a novel, minimally invasive balloon catheter design that enables localized organ perfusion (e.g., to the renal arteries) with only minimal disruption of aortic blood flow or requiring renal cannulation. The balloon catheter can be placed at the bedside and remain indwelling for the duration of the procedure or ICU stay. It can be also placed while in the emergency room or during resuscitation, allowing the medical team to treat the renal system in isolation from other organs (for example, permitting pulmonary edema to be controlled without increasing renal insufficiency). The use of the perfusion system of the invention will allow specific subsets of patients to recover from different conditions without risking irreversible renal damage.

As will be apparent to those skilled in the art, patients in risk for ARF/ARI, or such undergoing major cardiovascular surgery (such as coronary bypass surgery or abdominal aortic aneurysm repair), may benefit from the invention if treated before, during and/or after the said procedure.

ECG or arterial pressure synchronization signal could be used to control timing of obstructing and blood displacement elements alike. Nevertheless, it also allows activation of the system in fixed rates that can also be set by the operator. This system offers substantial benefits like:
a) easy reversible therapy - once the kidney has recovered and the ischemic episode is over, balloon catheter can be removed easily and safely, like any ordinary percutaneous catheter;
b) the risk of limb ischemia is minimized - since the device described in this invention is using a rather small diameter catheter (preferably less than 7 Fr -French Gauge-), that may well stay indwelling for an extended period of time, presenting no risk of putting the limb in danger of loss due to obstruction of blood flow at the groin artery;
c) ease of use - the straightforward insertion technique typically "Seldinger" or "modified Seldinger" technique (which is a most commonly used technique in the medical practice for the insertion of catheters into blood vessels). After a short in-service session, medical team will be able to handle all operational elements and troubleshooting; and
d) complementary, not competitive, with other devices or procedures - the simple small sized indwelling balloon elements will not interfere with other treatment or diagnostic modalities, including local drug delivery or required percutaneous interventions.

More particularly, in DRP applications the perfusion system of the invention allows provision of an easy and reversible therapy. Once the kidney has recovered and the ischemic episode is over, balloon catheter can be removed easily and safely, like any ordinary percutaneous catheter.

In addition, using the perfusion apparatus of the invention in DRP applications imposes significantly reduced risks compared with extensively used indwelling devices , since DRP is of much smaller sizes, even if compared to the standard Intra Aortic Balloon.

While the embodiments of the device of the present invention described herein above are particularly suitable for renal perfusion (DRP) the sequence of balloons/segments and/or direction of inflation/deflation could be modified for other specific applications, whether aimed at augmenting blood perfusion to a specific area or organ (including liver, heart, brain, limb etc.) or in order to enhance blood drainage from such areas or organs. Such applications could be in order to provide any such organ or area with more blood (oxygen and nutrition) and/or in order to assist in removing of blood (volume, fluids etc') from any such organ or area (e.g. reduce the afterload against which the heart contracts).

For example, Fig. 8 demonstrates a general embodiment of the invention wherein a sequence of numerous balloons are arranged to achieve a sequence of balloon cycles (inflation/deflation) which starts from the distal and proximal balloons, **81a** and **83a,** and proceeds via intermediate balloons **81b** and **83b,** and any additional intermediate balloon(s) which may be similarly connected to said intermediate balloons, towards the center of this balloon arrangement. The inflation/deflation of the proximal balloons **81** is effectuated via side opening **88** formed on the inflation/deflation channel **84** of the catheter **85,** the inflation/deflation of the distal balloon is effectuated via the opening at the distal end of channel **84,** and the inflation/deflation of the intermediate balloons **(81b** and **83b,...)** is effectuated via slender-passage elements **82** sequentially connecting their interiors to the interiors of the proximal or distal balloon, respectively. As was previously described, these slender-passage elements are introducing delays in the inflation deflation sequence. Therefore, the deflation sequence will also start in the deflation of the distal and proximal balloons, **81a** and **83a,** and proceeds with sequential deflations of the intermediate balloons, **81b** and **83b,** and any additional intermediate balloons similarly connected to.

Of course, other sequences of balloons arrangements may be achieved to provide, for instance, upward or downward sequence of balloons inflations/deflations, or with the used of additional conduits to connect the interiors of intermediate balloons to achieve combinations thereof, all by utilizing a single inflation/deflation channel to activate more than one balloon configuration (additional lumens could also be used, whether in order to facilitate more balloon configurations on the same catheter or in order to enable passage of other devices, like a guide wire or ultra sound prob, or in order to withdraw blood samples, infuse drugs or other fluids or take various measurements such as blood gas or pressure).

### EXAMPLE

In this example various parameters of the catheter were tested in simulation of a three balloon catheter arrangement (e.g., Fig. 2) in a patient of 70 bpm heart rate and a blood pressure of 60 mmHg. The inflation pressure (shuttle gas) used in this simulation was 100 mmHg and the length of the catheter shaft (from pump to first balloon in line) was 500 mm. The internal diameter of the simulated catheter shaft was 5 Fr, and the volume of the simulated proximal balloon volume 2 cc, the volume of the simulated displacement balloon was 6cc, and the volume of the simulated distal balloon was 2 cc. The internal diameter of the slender-passage element (between proximal and distal balloons) was 5Fr and the length of the conduit connecting between the distal and displacement balloon was 2mm long and its internal diameter 5Fr.

The simulation results were as follows:
- Proximal balloon filling time - 0.0233 Sec;
- Distal balloon filling time - 0.02453 Sec;
- Displacement balloon filling time - 0.0868 Sec with an overlap of 0.60 cc which equals to about 10%;
- A total filling time of 0.13469417 Sec;
- Blood displacement of - 0.38 cc/Min.

Those skilled in the art will also appreciate that perfusion system of the invention is easy to use due to its straightforward operation, which is preferably conducted utilizing a common insertion technique and familiar console, thus avoiding the need for specially trained personnel, like technicians or perfusionists to manage the system or monitor the patient.

The above examples and description have of course been provided only for the purpose of illustration, and are not intended to limit the invention in any way. As will be appreciated by the skilled person, the invention can be carried out in a great variety of ways, employing more than one technique from those described above, all without exceeding the scope of the invention.

## Claims

1. A balloon catheter comprising an elongated conduit adapted for insertion into a blood vessel, two or more balloons associated with said elongated conduit, and one or more additional conduits connecting the interior of two or more of said balloons, wherein said elongated conduit comprises two inflation/deflation channels which are suitable for transferring an inflation medium into and out of the balloons, and wherein said balloons and conduits are arranged such that said balloons may be inflated and deflated in a predetermined sequence.

2. The balloon catheter according to claim 1, **characterized in that** a first inflation/deflation channel connects to the interior of a proximal balloon and a second inflation/deflation channel connects to the interior of a distal balloon, and wherein an additional conduit connects the interior of said distal balloon and the interior of a central balloon situated between, and in proximity to, said proximal and distal balloons, and wherein said balloons are affixed to the distal portion of said elongated conduit.

3. The balloon catheter according to claim 1, **characterized in that** a first inflation/deflation channel connects to the interior of a proximal balloon and a second inflation/deflation channel connects to the interior of a distal section of a distally placed two-section balloon, and wherein the proximal section of said two-section balloon is arranged in proximity to said proximal balloon, and wherein an additional conduit connects the interior of said distal section and the interior of said proximal section, and wherein said balloons are affixed to the distal portion of said elongated conduit.

4. A balloon catheter according to claim 2, wherein the distal balloon is arranged such that there is a gap between said distal balloon and said central balloon.

5. A balloon catheter according to claim 2, wherein the proximal balloon is arranged such that there is a gap between the proximal balloon and the central balloon.

6. A balloon catheter according to claim 2, wherein the central balloon is enclosed in the interior of a hollow confining element, wherein said confining element comprises side apertures.

7. A balloon catheter according to claim 3, wherein the proximal section of the two section balloon is enclosed in the interior of a hollow confining element, wherein said confining element comprises side apertures.

8. A balloon catheter according to claim 2, wherein the distal tip of the elongated conduit extends beyond the distal end of the distal balloon, and wherein the interior of said distal balloon is connected to the interior of said elongated conduit via a side opening, and wherein said balloon catheter further comprises an additional lumen and a stiffening wire within said lumen.

9. A balloon catheter according to claim 3, wherein the distal tip of the elongated conduit extends beyond the distal end of the distal section of the two section balloon, and wherein the interior of said distal section is connected to the interior of said conduit via a side opening, and wherein said balloon catheter further comprises an additional lumen and a stiffening wire within said lumen.

10. A balloon catheter according to claims 8 or 9, further comprising a distal end section of the stiffening wire is manufactured from an elastic material in a coiled shape, such that when said distal end section of said wire exits the passage in which it is contained via a distal opening of said passage, the coiled shape of said wire is restored.

11. The balloon catheter of claim 2 or 3, wherein at least one of the balloons is manually and/or separately inflated via the appropriate channel.
